# EUROPEAN PATENT SPECIFICATION

(11) **EP 4 251 058 B1**
(45) Date of publication and mention of the grant of the patent: **09.09.2026**
(21) Application number: 21739321.4
(22) Date of filing: 01.07.2021
(51) Int. Cl.: A61B 8/00

(54) **ULTRASOUND COUPLING SYSTEM**
ULTRASCHALLKOPPLUNGSSYSTEM
SYSTÈME D'ACCOUPLEMENT À ULTRASONS

(30) Priority: 27.11.2020 EP 20210320
(43) Date of publication of application: 04.10.2023
(73) Proprietor: Aison Technologies AG, 8121 Benglen (CH)
(72) Inventor: KÄLIN, Philipp, 8121 Benglen (CH); BOROWKA, Sophia, 8121 Benglen (CH); KALLUNKATHARIYIL, Jinesh, 8121 Benglen (CH); ANDRICH, Luca, 8121 Benglen (CH)
(74) Representative: Frenkel, Matthias Alexander
(86) International application number: PCT/EP2021/068120
(87) International publication number: WO 2022/111866

(56) References cited:
- JP-A- H0 984 787
- US-A- 5 273 027
- US-A1- 2008 161 693
- US-A1- 2017 202 538
- US-A1- 2017 303 894

## Description

The present disclosure generally relates to an ultrasound coupling system for coupling an ultrasound transducer to a body part to be examined. In particular, the present disclosure relates to an ultrasound coupling system which comprises a stand-off holder for an ultrasound transducer and which is configured to removably mount a stand-off cushion which has the function of a coupling medium for guiding the ultrasound waves to the body part to be examined.

Ultrasound is one of the big versatile medical technologies to image the inside of the human or animal body. With this technology, an analysis of the anatomy, physiological states, tissue characteristics, etc. of a human or animal body are possible, which can eventually lead to a diagnosis of an injury or disease. Ultrasound can also be used for therapeutic purposes, for example, to treat an inflammation after excessive physical exercise or an injury.

The technology itself is a contact technology. Almost all ultrasound waves are reflected by air instead of making their way into the body when the ultrasound sensor and the body surface to be examined are not in absolute contact with each other. For this reason, a contact gel is used as a couplant or coupling media between the ultrasound transducer and the body to be examined. This will reduce the acoustic impedance and reflections. In some cases, ultrasound gel is not enough to grant full contact between the ultrasound sensor and the body because the body has diverse anatomical shapes, including troughs and ridges for which a huge amount of ultrasound gel is necessary to guarantee optimal contact. However, because of the ultrasound gel's high water content, the gel acts similar to a liquid, which means that it melts out of shape, or in other words, does not keep a firm shape but rather expands and drips like a liquid. One alternative solution is to use a water bath, where the animal or patient places their body part into it. This works well for hands, but harder for any bigger anatomical part (e.g. full leg or torso or head of a baby). Further, a water bath has the downside that it needs to be cleaned thoroughly to prevent any bacteria from staying inside the water bath and that the ultrasound transducer needs to be covered by a water-proof cover, which needs to contain a couplant medium to allow for an ultrasound transmission.

Another type of coupling medium is a stand-off pad. Different kinds of stand-off pads are known. For example, from document US 4 796 632 B a stand-off adapter for an ultrasound transducer is known which includes two compression-moulded parts each with a thin coupling surface formed at one end thereof during the compression moulding process. A space between the two coupling surfaces is filled with ultrasound gel. From US 6 139 502 B a handle housing for an ultrasonic transducer probe is known which comprises a base to which a stand-off pad filled with a fluid may be affixed thereby forming a fluid-impermeable seal. Document US 7 029 446 B2 discloses a stand-off holder and a stand-off pad for an ultrasound transducer, wherein the stand-off holder is configured as an elastic sock with a gel pad insert as the stand-off pad cut out according to the shape of the transducer and inserted into an opening of the elastic sock at an end portion thereof. Document US 6 132 378 B discloses an ultrasound transducer cover, where a stand-off pad is created by filling a space between two polyurethane sheets, which are bonded to a hole at the bottom of the ultrasound cover to close it from the bottom, with a liquid or gel. US 2017/202538 A1 discloses an ultrashield which dispenses with the need for an external couplant due to an outer surface body contact layer with openings for release of a couplant provided in an intermediate layer of the ultrashield. Portions of a probe contact layer form side walls which are configured to extend up and along a housing of a probe which include an adhesive. US 2017/303894 A1 discloses an ultrasound coupler having a solid coupler body with a cavity so as to conform to the end of a probe. The coupler may have a probe attachment member with an upwardly extending sidewall forming a perimeter of the cavity. The probe attachment member may comprise an adhesive on an inner surface thereof. JP H09-84787 discloses an ultrasonic probe water bag apparatus where a storage bag loaded with water may be removably fixed to a frame, which again may be detachably attached to a machine frame of an ultrasonic diagnostic apparatus.

There is a need to provide an ultrasound coupling system which is adapted to overcome problems related to the known systems. In particular, there is a need to provide an ultrasound coupling system with a stand-off pad having a maximum contact surface which optimally adapts to all kinds of body surfaces and body shapes and having a configuration independent from the configuration of the ultrasound transducer to be coupled with, while allowing an easy handling of an ultrasound transducer coupled to the ultrasound coupling system.

According to an aspect, an ultrasound coupling system for coupling an ultrasound transducer to a body surface to be examined is provided which comprises a stand-off holder for receiving the ultrasound transducer. The stand-off holder comprises a frame section with a central aperture, wherein the frame section comprises a fixation member for removably mounting a stand-off cushion to the stand-off holder. The central aperture is configured to accommodate an end portion of the ultrasound transducer comprising an end surface, and the frame section is configured such that the end surface contacts the stand-off cushion through the central aperture in a state where the stand-off cushion is removably mounted to the frame section and the end portion of the ultrasound transducer is accommodated in the central aperture.

The ultrasound coupling system of the present disclosure may be used in a variety of medical application fields. For example, the ultrasound coupling system may be used for musculoskeletal ultrasound imaging (e.g. Achilles tendon, hands, feet, arms, armpits) and for ultrasound imaging of small parts (e.g. ear, nose, neck) and of pediatrics (e.g. head, hip). The ultrasound imaging system may be further used in the vascular medicine. Further, the ultrasound coupling system may be used both for diagnostic and therapeutic purposes. Furthermore, the ultrasound coupling system may also be used for veterinarian ultrasound.

Furthermore, the ultrasound coupling system can be used with all kinds of ultrasound transducers. Examples of transducers are sector, array, convex, 3D/4D, micro-convex, linear or pencil transducers. The coupling system may be also used with large transducers used for veterinarian applications.

The frame section provides an interface between the ultrasound transducer, in particular between an end portion of the ultrasound transducer which may comprise an acoustic window, and the stand-off cushion. Thus, the stand-off holder is configured to couple the ultrasound transducer and the stand-off cushion, so that the stand-off cushion moves with moving the ultrasound transducer. Hence, a user only needs a single hand for operation.

The central aperture which is formed in the frame section may be configured as a through hole. Alternatively, the central aperture may form from an open frame. In a mounted state where the ultrasound transducer is received in the central aperture and the stand-off cushion is removably fixed to the frame section, the end surface of the ultrasound transducer which may comprise the acoustic window contacts an outer surface of the stand-off cushion. In particular, the stand-off cushion and the end portion face each other in the central aperture. The stand-off cushion may be mounted to a lower side of the frame section and the ultrasound transducer may be received in the central aperture from an upper, opposite side of the frame section. The central aperture has a fixed depth dimension and the end portion may be inserted in the central aperture so as to extend over the whole depth dimension. The end portion may also protrude beyond the central aperture so that it is immersed in the stand-off cushion. It may be immersed into the cushion up to several centimeters. The stand-off cushion has an upper surface facing the frame section and may be mounted so as to not protrude into the central aperture. Alternatively, the stand-off cushion or a part of the stand-off cushion may slightly protrude into the central aperture.

The fixation member is configured such that the stand-off cushion to be mounted may be a stand-alone cushion, that means a separate means that provides the function of a cushion also without being mounted to the frame section. Thus, the fixation member does not provide any sealing function to the stand-off cushion. The fixation member may be any fixation member that is comfortable to handle while providing a removable fixation. The fixation member may be configured to cooperate with a corresponding fixation member of the stand-off cushion, e.g. by latching, snapping, clamping etc. The fixation member may be fixedly connected to the stand-off holder, in particular to the frame section of the stand-off holder. Thus, the fixation member may be a fixed part of the stand-off holder. It may be integrally formed with the stand-off cushion. It may be connected so as to be located on an exterior surface of the stand-off holder. In that way the stand-off cushion may be mounted to an exterior side of the frame section. The fixation member may project beyond the frame section, in particular may laterally extend beyond the frame section.

The stand-off holder itself and the fixation member of the stand-off holder may be made of a material that can be easily cleaned, e.g. a biocompatible material.

The stand-off holder may accommodate different ultrasound transducers. For an optimal scanning experience and ultrasound images, the probe holder may come in different sizes to avoid any surplus material that could disturb the intuitive scanning process. Alternatively, for increased convenience and better usability, the probe holder may come in a single size for most transducers to avoid any time loss during the mounting process.

In an implementation of the first aspect, the fixation member is configured to removably mount the stand-off cushion to an exterior side of the frame section, in particular so as to laterally extend beyond the frame section. The stand-off cushion may be mounted to the exterior side so as to contact the end surface of the end portion with an upper surface. The stand-off cushion may be located completely outside the central aperture or may slightly protrude into the central aperture with a part of the upper surface. Thus, the coupling system allows mounting a stand-off cushion to all kinds of ultrasound transducers having varying shapes. Since the stand-off cushion may be mounted to an exterior side of the frame section, a dimension of the stand-off cushion is independent from a corresponding dimension of the central aperture. This applies both for a width dimension of the cushion as well as for a height dimension of the cushion. Thus, for example, the stand-off holder with the fixation member may be configured so that a dimension of the stand-off cushion is independent from a corresponding dimension of the central aperture, in particular of the dimensions of the stand-off holder. In particular, the cushion may laterally, i.e. in a width direction, project beyond the frame section. Furthermore, also the further characteristics of the cushion may be chosen independent from the configuration of the stand-off holder. In particular, a highly flexible stand-off cushion with a sufficient height dimension thereby being sufficiently flexible in the height dimension may be mounted for enabling to bridge over different anatomical structures like the Achilles tendon, the nose, the knuckles of the hand, the lateral and medial sides of the wrist, or even, if considered useful, inner organs. Thus, a contact surface between the cushion and the body part, and thus also between the ultrasound transducer and the body part, is maximized.

In a further implementation, the stand-off holder may be formed of a rigid material. The rigid material may be a plastic material, for example, in particular a biocompatible plastic material.

In a still further implementation, the stand-off holder may comprise a supporting section configured to support the ultrasound transducer accommodated in the central aperture, wherein the supporting section extends from the frame section in a direction perpendicular to an aperture plane of the central aperture. The aperture plane may be a plane that extends over the whole aperture and covers the whole aperture. The direction perpendicular to an aperture plane of the central aperture may be a longitudinal direction of the stand-off holder. Thus, the supporting section may be provided for supporting an upper portion of the ultrasound transducer adjoining the end portion. The supporting section may partially enclose the ultrasound transducer. Alternatively, the supporting section may completely enclose the respective part of the ultrasound transducer.

For example, in an embodiment the supporting section may comprise a supporting plate extending from the frame section in a direction perpendicular to the aperture plane of the central aperture. The supporting plate may be configured to support the ultrasound transducer along a longitudinal direction of the ultrasound transducer.

In the same embodiment or in a further embodiment, the supporting section may comprise a holding mechanism configured to removably hold the ultrasound transducer. The holding mechanism may be any kind of user-friendly releasable positive-locking, non-positive locking or a combination of positive-locking and non-positive-locking holding mechanism such as e.g. a latching, snapping and/or clamping mechanism. In that way, the stand-off holder can be used with different ultrasound transducers by simply releasing the holding connection and inserting and fixing another ultrasound transducer. The holding mechanism may, for example, comprise a holding bracket, in particular a clamping bracket, configured to hold a portion of the ultrasound transducer adjoining the end portion. Thus, an ultrasound transducer may be simply mounted by inserting and, optionally, clicking the ultrasound transducer in the holding mechanism. The ultrasound transducer may be additionally or alternatively held by one or more other holding means such as e.g. a band extending around the ultrasound transducer and the supporting section in a circumferential direction of the ultrasound transducer.

In a further implementation, the ultrasound coupling system further comprises a stand-off cushion comprising a flexible material and configured to be removably mounted to the frame section. The flexible material may be an elastomer material. For example, the stand-off cushion may comprise silicone. Additionally or alternatively, the stand-off cushion may comprise natural rubber (latex), polyisoprene, TPU (thermoplastic polyurethane), aromatic polyether polyurethane, TPE (thermoplastic elastomer) and/or hydrogel. Due to the flexible material the stand-off cushion may have flexible properties, in particular in a height direction of the stand-off cushion when moving over a body surface. In this way, the stand-off cushion may optimally adapt to and bridge over all kinds of surfaces and shapes of body portions thereby maximizing a contact area between the body and the cushion and thereby a measuring area captured by the ultrasound transducer. Further, by changing the pressure on the stand-off cushion, the height of the stand-off cushion can be adjusted manually and flexibly. In that way, it may be possible to dynamically adapt the focus in the ultrasound image. Typical dimensions of a stand-off cushion that will fit most ultrasound transducers are 7 cm for a length dimension and 2 cm for a width dimension. For large ultrasound transducers, typical dimensions are 7 cm for a length dimension and 4 cm for a width dimension.

The flexible material or materials may be combined with less flexible materials that allow for a good transmission of the ultrasound waves, for example plexiglass. In this manner, specific parts of the stand-off cushion may be stabilized, for example a top portion of the stand-off cushion, where the stand-off holder gets in contact with the cushion surface. Due to the stabilization and thereby minimization of folds that would lead to air holes, the stand-off cushion may, in addition to providing a safe minimal distance between the ultrasound transducer and the body part to be examined, e.g. increase the reliability in a robotic steering of the ultrasound transducer.

The shape of the stand-off cushion may be a round convex form, similar to a squeezed sphere, or a pillow or cushion. It could also have a bar- or ingot-shaped form. The thickness of the stand-off cushion may be below 15mm, such that the stand-off cushion does not fold to the sides during usage with a transducer of exactly the same or bigger size than the stand-off cushion. Furthermore, the shape may be chosen so as to facilitate a cleaning of the stand-off cushion, in particular if the cushion is a reusable cushion. The cushion may be also a disposable. It may be sterilized to be used on open wounds or inner organs, but also may be non-sterile for the use on any part of the skin.

According to an aspect of the further implementation, an outer dimension of the stand-off cushion in a width direction of the stand-off cushion is different from a corresponding dimension of the central aperture in a width direction of the central aperture, in particular is greater than a corresponding dimension of the central aperture in a width direction of the central aperture. A corresponding dimension means a dimension in the same direction when the stand-off cushion is mounted to the stand-off holder. Thus, in other words, the stand-off cushion may have a different shape than the central aperture and also than the frame section, so that the shape of the stand-off cushion may be chosen independent from the shape and configuration of the aperture and the frame section. Thus, the stand-off cushion may, for example, laterally extend beyond the frame section when the stand-off cushion is removably mounted to the frame section.

In an embodiment, the stand-off cushion may comprise a fixation member configured to cooperate with the fixation member of the stand-off holder for removably mounting the stand-off cushion to the stand-off holder, in particular to an exterior side of the stand-off holder. The cooperating fixation member may be any kind of releasable fixation member, such as a snapping, clamping or latching means. The cooperating fixation member may form a mounting means. For example, the mounting means may comprise a rigid ring provided in the interior of the stand-off cushion and a mounting structure such as mounting clips attached to the plastic ring. The mounting structure such as the clips may cooperate with a corresponding mounting structure provided at the frame section, for example mounting clip holders. The corresponding mounting structures may be additionally fixed by (a) further fixation member(s). The stand-off cushion may be also provided with flaps, into which extensions (cushion holders) provided at the frame section may be inserted. The stand-off cushion may also have a separate extrusion forming a ring, which cooperates with the cooperating fixation member of the holder. In other words, the ring inside the cushion may also be replaced by a similarly shaped extrusion on the cushion envelope.

In an implementation, the stand-off cushion may comprise an envelope which forms a closed hollow which is filled with a liquid, gel-like and/or relatively soft material, wherein the envelope comprises a flexible material. "Relatively soft" means a softness relative to the flexible material of the envelope, i.e. the relatively soft material of the inner filling has a lower hardness than the flexible material of the envelope. The "relatively soft" material also may be a flexible material. The flexible material may be, for example, any material that has been listed above with respect to the flexible material of the overall stand-off cushion. In particular, the flexible material may be an elastomer material. For example, the flexible material of the envelope may be a silicone, and the filling material may also comprise or consist of a silicone, but which is softer than the silicone of the envelope (liquid or very soft silicone). In another embodiment, the filling material may include water, vegetable oil, ultrasound gel, water based gels, mineral oil, oil-based gels, glycerin, hydrogel, liquid paraffin or any other material that has a similar acoustic impedance and low attenuation coefficient. In still another embodiment, the filling material may include ultrasound gel or water and the outer envelope may be made of or comprise an aromatic polyether polyurethane. Aromatic polyether polyurethane is characterized by a high elasticity and an excellent hydrolytic stability and barrier function. An outer envelope made of or comprising an aromatic polyether polyurethane may have a material thickness of 20 to 25 µm.

In an alternative implementation, the stand-off cushion may comprise an envelope with an opening, wherein the envelope forms a hollow and is filled with a gel-like and/or relatively soft material. The opening may be located so that it faces the end surface of the ultrasound transducer in a state where the stand-off cushion is mounted to the frame section and the end portion of the ultrasound transducer is accommodated in the central aperture. The envelope may comprise a flexible material.

As an example embodiment of the alternative implementation, the stand-off cushion may only comprise one membrane instead of two membranes thereby forming a hollow that comprises at least one opening. The opening may be provided in an upper surface of the stand-off cushion being configured to be contacted by the ultrasound transducer, wherein the upper surface may be covered by the second membrane in an implementation with two membranes and a closed hollow. The open, upper surface may be located in the central aperture of the frame section when the stand-off cushion is mounted to the stand-off holder. In particular, the open, upper surface may be at least partly exposed through the central aperture of the stand-off holder. In that way, the ultrasound transducer when being accommodated in the central aperture of the frame section may contact the open surface of the stand-off cushion, i.e. may contact the filling material of the stand-off cushion. For ensuring a good fitting of the sizes of the central aperture and the ultrasound transducer, the frame section may comprise telescopic elements that allow for a flexible change in the size of the frame section. Furthermore, the frame section may comprise a locking mechanism configured to lock the telescopic elements with respect to each other, thereby keeping the frame section in the desired size once the fitting frame section size has been found.

As a further example embodiment of the alternative implementation, the stand-off cushion may comprise two (or more membranes) and at least one membrane of the two (or more) membranes has at least one opening. For example, a cover membrane or foil of the stand-off cushion may comprise an opening. The opening may be shaped like a slit or slot hole. The opening may not be closed during usage of the stand-off cushion. The opening may be located in the central aperture of the frame section when the second modified stand-off cushion is mounted to the stand-off holder. Furthermore, the size of the opening may be chosen to be the same size as the size of the ultrasound transducer, i.e. the end surface of the end portion of the ultrasound transducer.

The envelope may be further stabilized by the insertion of less flexible materials that allow for a good transmission of the ultrasound waves, such as e.g. plexiglass. The envelope may be also stabilized only in selected regions. For example, a top portion of the envelope being adjacent to the frame section when the stand-off cushion is mounted to the frame section may be configured with a greater stiffness than a bottom portion of the envelope being further away from the frame section when the stand-off cushion is mounted to the frame section. The greater stiffness may be achieved by a greater envelope wall thickness in the selected region and/or by using (additionally or alternatively) another or the same material with less flexibility for the envelope in the selected region.

In another embodiment, the stand-off cushion may comprise an opening for selectively connecting a pressure-regulating mechanism. The pressure-regulating mechanism may be, for example, a tube with a valve or a single tube. The opening may be used to fill the cushion with purified water. A pressure regulation may occur by water flowing out of the stand-off cushion when the pressure exerted on the body part is too high. The opening may be closable by an appropriate cap or plug, if no pressure regulation of the stand-off cushion on the body surface should be achieved.

According to another aspect of the present disclosure, an ultrasound coupling system for coupling an ultrasound transducer to a body surface to be examined is provided which comprises a flexible stand-off cushion, and a tab connected to the stand-off cushion and having an adhesive region for coupling the stand-off cushion to the ultrasound transducer. In the other aspect, the ultrasound coupling system does not comprise a separate stand-off holder, but the tab assumes the coupling function of the stand-off holder. The tab may comprise two or more tabs. Further, the tab or the tabs may comprise one or more adhesive regions. The tab may be permanently connected to the stand-off cushion. Thus, the ultrasound coupling system with the stand-off cushion and the tab may be a disposable. The ultrasound transducer may be placed on an upper surface of the stand-off cushion and the tab may be bent in a direction toward the ultrasound transducer so that the adhesive region contacts an outer surface of the ultrasound transducer, thereby adhesively connecting the stand-off cushion to the ultrasound transducer. The tab may be flexible, allowing an adaptation to a various kinds of ultrasound transducer sizes and shapes. The adhesive region may have portions with greater extensions, for example in a width direction of the stand-off cushion, forming adhesive points configured to tightly glue the stand-off cushion to the ultrasound transducer. The adhesive region may be provided in any manner known to the skilled person. For example, the adhesive region may be provided by an adhesive foil, wherein the adhesive foil may be a one-sided adhesive or a two-sided adhesive. As a further example, the adhesive region may also be provided by applying a glue on defined portions of the tab. The fixing of the stand-off cushion by means of the adhesive tab may be removable. Thus, the stand-off cushion may be removed from the ultrasound transducer without leaving any adhesive residue.

The stand-off cushion of the other aspect provides the same advantages as the stand-off cushion of the first described aspect. In particular, inter alia, the ultrasound coupling system provides a stand-off cushion having a maximum contact surface which optimally adapts to all kinds of body surfaces and body shapes and having a configuration independent from the configuration of the ultrasound transducer to be coupled with, while allowing an easy handling of an ultrasound transducer coupled to the ultrasound coupling system. Thus, the other aspect provides an alternative aspect to the first described aspect.

In an implementation of the other aspect, the flexible stand-off cushion may have an envelope forming a hollow and the tab may be integrally formed with the envelope. The tab may be integrally formed with the envelope of the stand-off cushion by attaching an upper envelope layer with a lower envelope layer at predefined areas where the upper and lower envelope layers extend beyond the stand-off cushion, preferably by welding the upper and lower envelope areas. For example, the upper and lower envelope layers may extend centrally beyond the stand-off cushion, i.e. beyond a long side of the stand-off cushion, for example, or laterally and centrally, beyond the stand-off cushion. In this implementation of the other aspect, the envelope layers may also be one-sided adhesive such that no additional adhesive is necessary.

In another implementation of the other aspect, the ultrasound coupling system may comprise a frame holding the stand-off cushion, wherein the tab may be connected to the frame. In the other implementation, the central stand-off cushion may be surrounded by and connected to a rigid frame, for example by welding. The tab may be two tabs which are connected to opposite sides of the frame. Furthermore, the tab or the tabs may be pivotally connected to the frame. An upper surface of the tab or tabs, i.e. a surface facing the ultrasound transducer when the ultrasound transducer is placed on the stand-off cushion, may comprise an adhesive region for adhesively connecting the stand-off cushion to the ultrasound transducer.

In a further implementation of the other aspect, the flexible stand-off cushion may comprise an envelope which forms a hollow having an opening and being filled with a gel-like and/or relatively soft material, wherein the opening is located so that it faces the end surface of the ultrasound transducer in a state where the stand-off cushion is coupled to the ultrasound transducer.

As an example embodiment of the further implementation, an upper envelope layer or cover of the stand-off cushion may comprise an opening. The opening may be shaped like a slit or slot hole. The opening may or may not be closed during usage of the stand-off cushion. The size of the opening may be chosen to be substantially the same size as the size of the ultrasound transducer, i.e. the end surface of the end portion of the ultrasound transducer.

As an alternative example embodiment of the further implementation, the stand-off cushion may only comprise a lower envelope layer instead of two envelope layers thereby forming a hollow that comprises at least one opening, i.e. the opening is caused by the lacking envelope layer. The opening may be provided in an upper surface of the stand-off cushion being configured to be contacted by the ultrasound transducer, wherein the upper surface corresponds to the surface to be covered by the second membrane or second envelope in an implementation with two membranes or two envelopes and a closed hollow. In that way, the ultrasound transducer when being fixed with respect to the cushion by means of the tab may contact the open surface of the stand-off cushion, i.e. may contact the filling material of the stand-off cushion.

According to a further aspect of the present disclosure, an ultrasound measurement system for guiding an ultrasound transducer comprising one of the above-described ultrasound coupling systems is provided. The ultrasound measurement system may comprise a robotic system which is configured to steer an ultrasound transducer accommodated in the ultrasound coupling system of the present disclosure, e.g. by screws or a clicking mechanism. The stand-off holder for the autonomous system should be configured to ensure a constant insertion angle (in all three directions in space) of the ultrasound transducer within the stand-off holder, to guarantee a reproducible scanning with the autonomous ultrasound system. Alternatively, the ultrasound measurement system may comprise a guiding system which is configured to manually guide an ultrasound transducer accommodated in the ultrasound coupling system along predetermined paths, thereby standardizing the ultrasound measurement, without any active motor controls. The guiding system may be, for example, a guiding rail system or a frame system to which the ultrasound coupling system may be coupled, thereby determining a fixed path for moving the ultrasound transducer. To achieve a constant insonation angle, which is different from the default 90° insonation angle, the stand-off holder may be fixed to the guiding system by an inclined block. Since the stand-off cushion is very deformable so that it bridges over all kinds of anatomical shapes, steering of a robot is simplified compared to known transducer adapters with only a thin membrane and/or a cushion that is not so flexible in the lateral and/or height direction.

According to a still further aspect according to the present disclosure, a method for manufacturing a stand-off cushion for use in an ultrasound coupling system comprising a stand-off holder is provided. The method comprises the steps of providing an envelope comprising a first elastomer material, the envelope defining an inner hollow having an opening, filling the inner hollow with a second elastomer material through the opening, wherein the second elastomer material has a lower hardness than the first elastomer material, closing the opening with a third elastomer material, and providing a fixation member for mounting the stand-off cushion to the stand-off holder.

The process steps do not necessarily need to be performed in the order as defined above. For example, providing the fixation member may be performed together with the step of providing an envelope. The first, second and/or third elastomer materials may be a silicone. The second elastomer material that is filled in may be a liquid or very soft elastomer material. After filling in, the first elastomer material may connect with the second elastomer material to a coherent mass via cross-linking. The closing of the opening may be performed directly after filling in the second elastomer material, for example by applying a foil comprising the second elastomer material, so that the second elastomer material may also connect with the third elastomer material, or the third elastomer material may be applied in a separate, later manufacturing step. After closing the opening, the envelope forms a fluid-tight housing for the inner elastomer material. The fixation member may comprise openings provided in the envelope. The fixation member may also comprise a ring arranged inside the inner hollow. The fixation member may also comprise a frame-like ring outside the inner hollow. Since the first elastomer material is harder than the second elastomer material, the envelope forms a stable shell for the second elastomer material.

The third elastomer material may be the same elastomer material as the second elastomer material. In particular, the third elastomer material may be the same material with the same hardness as the second elastomer material. The third elastomer material closing the opening may be applied after hardening of the second elastomer material.

In an implementation of the method, the step of providing the envelope may include mould casting the envelope with the first elastomer material and subsequently hardening the first elastomer material before filling in the second elastomer material. Alternatively, the step of providing the envelope may also comprise gluing several foils together so as to achieve a desired shape of the envelope.

Further details, advantages and aspects of the present disclosure will become apparent from the following embodiments taken in conjunction with the drawings, wherein:
- Fig. 1: shows in a perspective view an ultrasound coupling system according to an embodiment of the present disclosure, with a stand-off cushion and an ultrasound transducer mounted to a stand-off holder;
- Figs. 2a to 2c: schematically show different perspective views of a stand-off holder according to another embodiment of the present disclosure;
- Fig. 3: schematically shows in a sectional view an embodiment of a stand-off cushion according to the present disclosure;
- Fig. 4: schematically shows in a perspective view a further embodiment of a stand-off cushion according to the present disclosure;
- Fig. 5: shows a flow diagram of a method for manufacturing a stand-off cushion for use in an ultrasound coupling system comprising a stand-off holder according to the present disclosure;
- Fig. 6: shows in a perspective view an ultrasound coupling system according to another embodiment of the present disclosure, with a stand-off cushion being intended to be adhesively attached to an ultrasound transducer; and
- Figs. 7a and 7b: show an ultrasound coupling system according to a still another embodiment of the present disclosure for adhesively attaching the stand-off cushion to an ultrasound transducer, wherein Fig. 7b shows in an enlarged view one tab of the two tabs of Fig. 7a having an adhesive region for connecting the cushion to an ultrasound transducer.

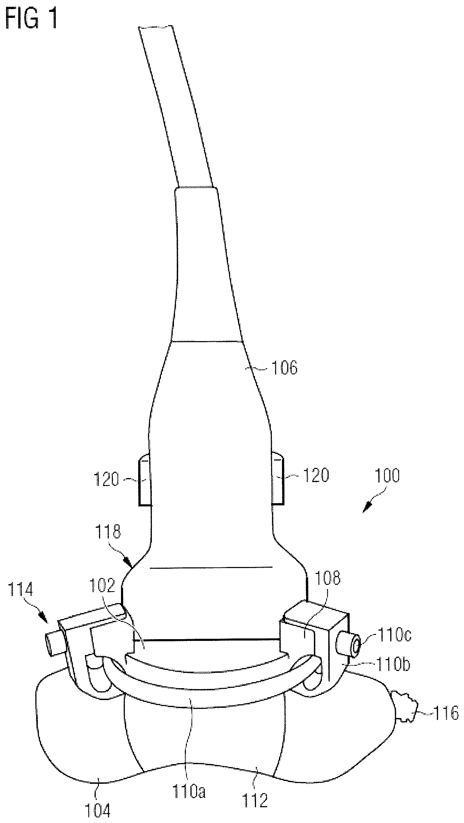

Fig. 1 shows a perspective view of an embodiment of the ultrasound coupling system 100 in a fully mounted form, which comprises a stand-off holder 102 and a stand-off cushion 104 mounted thereto, wherein an ultrasound transducer 106 is accommodated in the stand-off holder 102 so as to be positionally fixed therein. The stand-off cushion 104 is removably mounted to the stand-off holder 102 by means of corresponding fixation members 108, 110 forming a mounting system.

The stand-off cushion 104 has an envelope 112 forming a closed space which is filled with a liquid, gel-like and/or relatively soft material. The stand-off cushion 104 is thus a stand-alone device, being autonomous from the stand-off holder 102 and the ultrasound transducer 106. As is seen in Fig. 1, the stand-off cushion 104 is mounted to an exterior side of the stand-off holder 102 to the stand-off holder 102, in particular to an exterior side of a bottom part of the stand-off holder 102, so as to be located exterior from the stand-off holder 102. Thus, the shape and size of the stand-off cushion 104 is independent from the shape and size of the stand-off holder 102 and an end portion of the ultrasound transducer 106, in particular from the bottom part of the stand-off holder 102. Thus, the stand-off cushion 104 may laterally extend beyond a frame section with a central aperture of the stand-off holder 102 (which will be described later). The stand-off cushion 104 may also have a height dimension being independent from any dimensions of the stand-off holder 102, such as a depth of the aperture, for example.

For mounting the stand-off cushion 104 to the stand-off holder 102, the stand-off cushion 104 has a fixation member 110 which is configured to cooperate with a corresponding fixation member 108 of the stand-off holder 102. In particular, in the present embodiment, the stand-off cushion 104 contains a plastic ring 110a arranged in the interior of the stand-off cushion 104 to which clips 110b are fixed through the envelope 112 of the stand-off cushion 104. The clips 110b are attached to clip holders 108 provided at the bottom part of the stand-off holder 102 by means of screws 110c. The plastic ring 110a, the clips 110b, the clip holders 108 and the screws 110c form a mounting system 114. The mounting system 114 is made of a stiff material that can be easily cleaned, e.g. a biocompatible plastic.

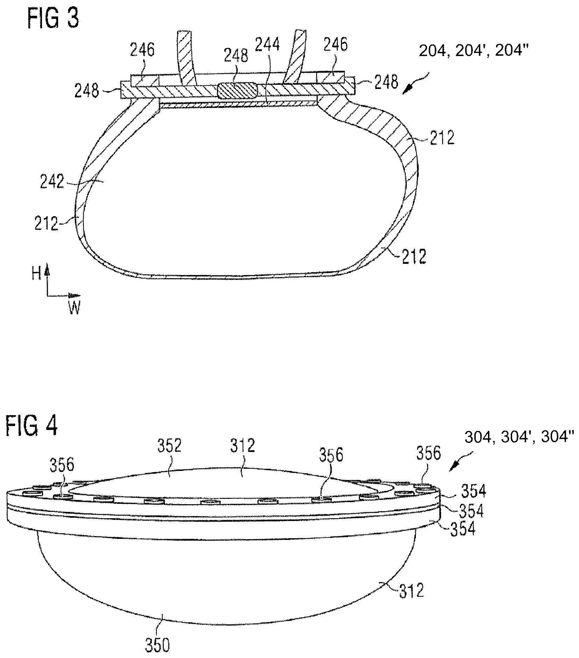

According to the present disclosure, the stand-off cushion 104 may be removably fixed to the stand-off holder 102 in any user-friendly way. For example, the clips 110b and the clip holders 108 may be configured so that they can be clipped together, for example by means of a snap mechanism or a latching mechanism, without the need of any screws. In a further alternative configuration, the screws 110c are replaced by pins. In a still further alternative configuration, the plastic ring 110a may be directly fixed to the stand-off-holder 102 by means of a fixation member provided at the stand-off-holder 102, for example by clipping the plastic ring 110a into clips provided at a stand-off-holder 102. In a still further alternative configuration the plastic ring may be replaced by a ring made of a harder elastomer having a profile that matches the opposite profile (made of a hard elastomer) on the stand-off holder. Further alternative configurations of removably fixing a stand-off cushion to a stand-off-holder will be described later with respect to Figs. 3 and 4 of the drawings.

The envelope 112 of the stand-off cushion 104 of Fig. 1 results from a mould casting process. However, the envelope 112 may be also formed by other manufacturing processes, for example by gluing several foils together so that a desired shape is achieved.

The stand-off cushion 104 contains an opening 116 on the right-hand side with respect to Fig. 1. The opening 116 may be used to fill the cushion with purified water. The opening 116 may be closed by an appropriate closure so that no pressure regulation of the stand-off cushion 104 on the body (part) surface is achieved. Alternatively, the opening 116 may be connected to a pressure-regulating mechanism (not illustrated in Fig. 1), such as a tube containing a valve or only a single tube, thereby achieving pressure regulation by water that flows out of the cushion 104 when a pressure is exerted on the skin or on the body part.

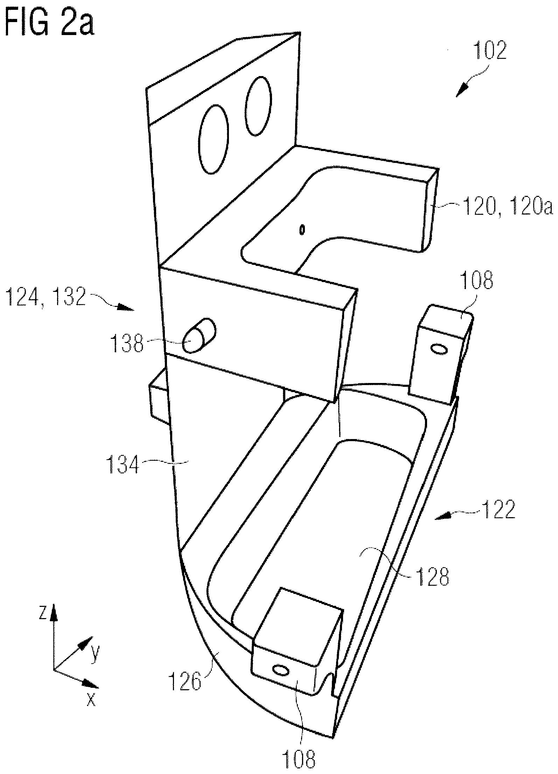

The stand-off holder 102 comprises a bottom part with a frame section for accommodating an end portion 118 of the ultrasound transducer 106 and a top part with a holding mechanism 120 for holding the ultrasound transducer 106 in the stand-off holder 102. In Fig. 1, the holding mechanism 120 are holding brackets into which the ultrasound transducer 106 is clicked. As is seen, the stand-off holder 102 does not form a closed housing for accommodating the ultrasound transducer 106, but is an open housing rather forming a holding framework than a housing enclosing the ultrasound transducer 106. The stand-off holder 102 is also maybe made of a stiff material that can be easily cleaned, e.g. a biocompatible material. An embodiment of a stand-off holder will be described in more detail with reference to Figs. 2a to 2c.

In the mounted state as depicted in Fig. 1, an end surface of the end portion 118 of the transducer 106 accommodated in an aperture (not seen in Fig. 1) formed in the bottom part of the stand-off-holder 102 directly faces the part of the stand-off cushion 104 that extends over the aperture so that the end surface, more particularly an acoustic window of the end surface, contacts the stand-off cushion 104.

An embodiment of a stand-off-holder 102 is shown in Figs. 2a to 2c of the drawings. The stand-off-holder 102 comprises a bottom part 122 configured to provide a defined coupling interface between the stand-off cushion 104 and the ultrasound transducer 106, and a top part 124 configured to stably hold the ultrasound transducer 106 in the stand-off holder 102.

The bottom part 122 comprises a frame section 126 with a central aperture 128 former therein. The central aperture 128 is configured as a through hole. The central aperture 128 is further configured to accommodate the end portion 118 of the ultrasound transducer 106 comprising an acoustic window. In particular, the central aperture 128 has dimensions that correspond to the outer dimensions of the end portion 118 of the ultrasound transducer 106 so that the end portion 118 may be received in the central aperture 128. The bottom part 122 or frame section 126 further comprises clip holders 108 for mounting the stand-off cushion 104. In the present embodiment, the frame section 126 comprises two clip holders 108, one at each lateral, front end of the frame section 126. The frame section 126 may also comprise any other number of clip holder 108 or clip holders 108.

A third clip holder 108 is provided at a back surface 130 of the stand-off holder 102, in particular at a back surface 130 of the top part 124 of the stand-off holder 102 which forms a supporting section 132 for further supporting the ultrasound transducer 106 accommodated in the central aperture 128. In the present embodiment, the supporting section 132 comprises a supporting plate 134 which extends from the frame section 126 in a direction perpendicular to an aperture plane of the central aperture 128, i.e. in a longitudinal direction (z-direction) of the stand-off-holder 102. The aperture plane is defined as a plane extending through the aperture 128 and extending over the whole aperture 128 so as to cover or close the aperture 128. Thus, in the geometry of Fig. 2a, the aperture plane lies in the xy-plane. In the embodiment of Figs. 2a to 2c, the supporting plate 134 has a flat, inner surface and a flat, outer surface with beveled edges 136. The third clip holder 108 is provided at a lower part of the supporting section 132 next to the frame section 126.

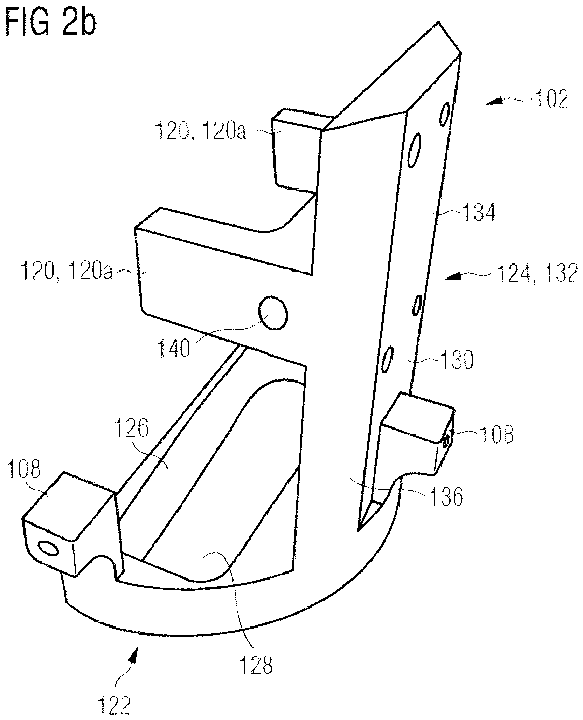

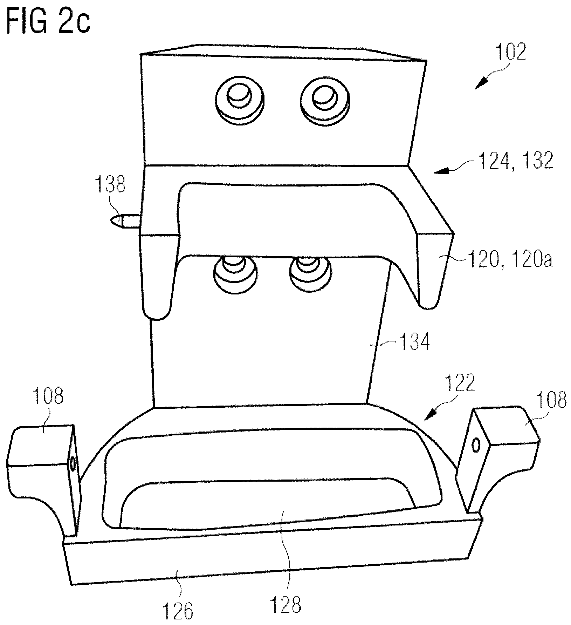

As described above with respect to Fig. 1, the supporting section 132 comprises the holding mechanism 120 for removably holding the ultrasound transducer 106, in particular an upper part of the ultrasound transducer 106 adjoining the end portion 118. In the present embodiment, the holding mechanism 120 is configured as a holding bracket 120a that partially extends in a circumferential direction of the stand-off holder 106 perpendicular to the longitudinal direction (z-direction). The ultrasound transducer 106 is received by being clicked into the holding bracket 120a, which then has the function of a clamping bracket. Additionally or alternatively, the ultrasound transducer 106 is tightened with a flexible band (not illustrated), which is guided around (i.e. in a circumferential direction) the holding bracket 120a and the ultrasound transducer 106 received therein. The flexible band may be fixed to the stand-off-holder 102 with a screw at an end portion of the flexible band, and the flexible band may be fixed to the stand-off-holder 102 at an opposite end thereof by means of a pin provided at the stand-off-holder 102 onto which a hole in the flexible band can be pulled. The pin 138 provided at the holding bracket 120a is seen in Figs. 2a and 2c of the drawings, and a hole 140 provided at an opposite portion of the holding bracket 120a for inserting the screw is shown in Fig. 2b of the drawings. Alternatively, the function of the flexible band can be taken over by a convex counterpart to the holding bracket. In other words, the ultrasound transducer 106 may also be tightened with a second holding bracket, forming a clamping bracket together with the holding bracket 120a. The two brackets may be connected by a hinge. The two brackets can close by means of a ratchet using the ratchet effect. In another alternative, the brackets can be closed by magnets. In another alternative, the holding section can contain a magnet.

The combination of the top part 124 and the bottom part 122 in the stand-off-holder 102 allows controlling the insonation angle during the ultrasound measurement or treatment process, which, for example, allows to standardize ultrasound measurements.

According to the present disclosure, it is also possible to only provide the frame section 126 (bottom part 122) without the top part 124. In that way, the frame section 126 holds the stand-off cushion 104, and the ultrasound transducer 106 can be inserted into the aperture without being supported by the supporting section. In this conventional way, a user may need two hands, one for holding the frame section with the stand-off cushion and the other for holding the ultrasound transducer in the central aperture.

Fig. 3 schematically shows a sectional side view of an embodiment of a stand-off cushion 204 according to the present disclosure. In particular, Fig. 3 shows a section through a central portion of the stand-off cushion 204. In addition, Fig. 3 shows a height direction H and a width direction W of the stand-off cushion 204. In the embodiment of Fig. 3, the envelope 212 in a top portion thereof (adjacent to the frame section 126 when the stand-off cushion 204 is mounted to the frame section 126) has a greater wall thickness than a bottom portion of the envelope 212 (being further away from the frame section 126 when the stand-off cushion 204 is mounted to the frame section 126). This results in a greater stiffness of the envelope wall in the top portion than the envelope wall in the bottom portion.

In the present embodiment, the envelope 212 is made of silicone. Also a hollow 242 formed by the envelope 212 is filled with silicone. Advantageously, the envelope 212 is made of soft silicone (e.g. shore A silicones), and the hollow 242 is filled with extremely soft silicone (e.g. shore 0 silicones). Generally, the silicone inside the envelope 212 is softer than the silicone forming the envelope. The filled envelope 212 is closed with a silicone cover 244, which may also be the soft silicone. The envelope 212 with the cover 244 forms a closed hollow 242 for the inner silicone.

Further in the present embodiment, the envelope 212 forms a collar 246 that longitudinally (i.e. in a height direction H of the stand-off cushion) extends beyond the silicon cover 244. In a circumferential direction of the collar 246, a plurality of mounting openings 248 may be provided in the collar 246. In the present embodiment, four mounting openings 248 arranged in a cross are provided. Into the plurality of mounting openings 248, a plurality of corresponding extensions provided at the stand-off holder, in particular at the frame section thereof, may be inserted for mounting the stand-off cushion to the stand-off holder, as is schematically illustrated in Fig. 3. The mounting openings 248 have a sufficient stability for holding the stand-off cushion 204 at the stand-off holder 102 since the collar 246 is made of the harder silicone and/or an increased wall thickness. Thus, in this embodiment, no inner plastic ring is provided.

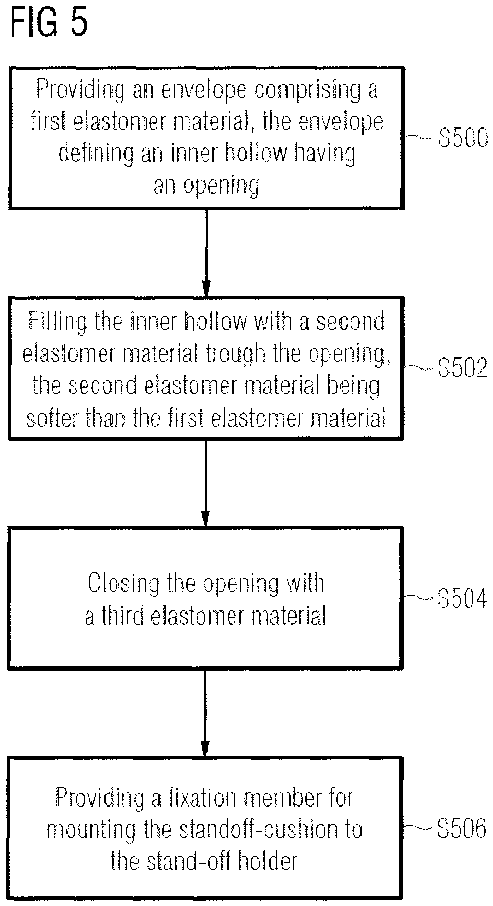

Fig. 5 shows a method how the stand-off cushion 204 of Fig. 3 may be manufactured. In step S500, the envelope 212 is provided comprising a first elastomer material. In Fig. 3, the first elastomer material is a silicone. The envelope 112 defines in inner hollow 242 with an opening.

In step S502, the inner hollow 242 is filled with a softer, second elastomer material, in Fig. 3 the extremely soft silicone, through the opening after the outer, soft silicone layer of the envelope is hardened. The inner soft, liquid silicone and the outer, hardened silicone may then connect, thereby avoiding the formation of air holes.

In step S504, the opening is closed with a third elastomer material thereby forming the cover 244. In Fig. 3, the cover 244 is made of the harder silicone of the second elastomer material and is applied in a subsequent manufacturing step as a thin protective layer, after hardening of the inner, soft silicone. Alternatively, the cover 244 may be directly applied after filling in the soft silicone, for example by applying a foil. After hardening and connecting, the outer silicone of the envelope and the cover and the inner silicone together form a coherent mass.

In step S 506, a fixation member for mounting the stand-off cushion to the stand-off holder is provided. In the embodiment of Fig. 3, the fixation member are the mounting openings 248. The mounting openings 248 can be created directly during casting with a mould, which is a cost-effective way, or, alternatively, may be provided after the casting process, e.g. by punching.

The greater stiffness of the envelope wall in the top portion compared to the stiffness of the envelope wall in the bottom portion improves a pulling of the stand-off cushion over the body surface or body part since the stand-off cushion is stable against shear stresses thereby better maintaining its shape and minimizing the risk of tearing.

Alternatively, instead of the casting process for the envelope, it is possible to first provide a very soft silicone cushion, which is then coated by a harder silicone to give it a stable cover, e.g. by dipping the soft silicone cushion into the harder silicone.

Thus, in this method, an envelope or a cover with a substantially uniform wall thickness is provided.

Fig. 4 shows another embodiment of a stand-off cushion 304 according to the present disclosure. The envelope 312 of the stand-off cushion 304 is made of two silicone foils, one silicone foil 350 forming a bottom, convex trough or hollow, and the other silicone foil 352 forming a top cover for the through or hollow. A frame 354 is provided for clipping the two silicone foils 350, 352 together thereby forming a closed, fluid-tight hollow. In the present embodiment, the shape of the frame 354 is round to minimize the size and impact of folds that are formed when inserting the bottom foil 350 into the frame 354. Further in the present embodiment, the frame 354 consists of three ring-shaped sub-frames, i.e. an upper sub-frame and two-lower sub-frames. The two lower sub-frames comprise an outer lower sub-frame and an inner lower sub-frame, wherein the outer lower sub-frame surrounds the inner lower sub-frame (i.e. the inner lower sub-frame has a smaller radius than the outer lower sub-frame). The upper foil 352 is clipped between the upper sub-frame and the two lower sub-frames, and the lower foil 350 is clipped between the outer lower sub-frame and the inner lower sub-frame. The sub-frames with the foils 350, 352 therebetween may be fixed together by a plurality of screws 356 which are inserted from the top to the bottom through corresponding openings provided in the sub-frames. In this embodiment, only the upper sub-frame and the outer lower sub-frame are screwed together. The inner lower sub-frame is kept in place because the upper sub-frame extends over the two lower sub-frames in width, and the outer lower sub-frame has an extrusion extending inside the inner lower sub-frame, or vice versa (like two rings clicked into each other). In this embodiment, the screws 356 are arranged around the whole circumference of the frame 354 and are regularly spaced. In an alternative embodiment, the screw holes can be replaced by pins and the upper frame and the outer lower frame may be welded together, for example by ultrasound welding.

Before fully tightening the foils together, an opening is left (via a few unscrewed screw holes) to fill the hollow with a liquid, gel-like or viscous material. Alternatively, after tightening the foils together, the closed hollow may be filled with a liquid, gel-like or viscous material by means of a tiny valve formed on the top foil (not illustrated in Fig. 4). In yet another alternative, a tiny valve is placed between the upper sub-frame and the lower sub-frames (not illustrated in Fig. 4), through which, after tightening the foils together, the closed hollow may be filled with a liquid, gel-like or viscous material.

The stand-off cushions 204, 304 of the embodiments of Figs. 3 and 4 each comprise two membranes that are attached so as to form a closed hollow. In particular, the stand-off cushion 204 of Fig. 3 comprises an envelope 212 and a cover 244 which form a closed hollow 242. The stand-off cushion 304 of Fig. 4 comprises two foils 350, 352 attached to the frame 354 so as to form a closed, fluid-tight hollow (Remark: 312 is the reference sign for the envelope). According to the present disclosure, the stand-off cushion may also only comprise one membrane instead of two membranes thereby forming a hollow that comprises at least one opening.

For example, the stand-off cushion 204 of Fig. 3 may be modified so as to comprise only the envelope 212 and to no longer comprise the cover 244. Analogously, the embodiment of Fig. 4 may be modified so as to comprise only the foil 350 forming the bottom trough or hollow and to no longer comprise the foil 352 forming the top cover. The such modified stand-off cushion 204', 304' according to a first modification forms an open hollow with an opening in an (with respect to Figs. 3 and 4) upper surface of the stand-off cushion. The open, upper surface of the modified stand-off cushion 204', 304' is located in the central aperture 128 of the frame section 126 when the modified stand-off cushion 204', 304' is mounted to the stand-off holder 102. In particular, the open, upper surface is at least partly exposed through the central aperture 128 of the stand-off holder 102. In that way, the ultrasound transducer 106 when being accommodated in the central aperture 128 of the frame section 126 contacts the open surface of the modified stand-off cushion 204', 304', i.e. contacts the filling material of the modified stand-off cushion 204', 304'.

The modified stand-off cushion 204', 304' with the open, upper surface has the advantage that potentially occurring reverberation artifacts may be reduced. Furthermore, the open, upper surface dispenses with the need to apply a coupling agent on the upper surface of the stand-off cushion, a coupling agent only has to be applied on the opposite, lower side of the stand-off cushion.

In order to avoid a spilling of the filling material through the opening, it is advantageous that the central aperture 128 of the stand-off holder 102 substantially fits the size of the ultrasound transducer 106, i.e. the end portion 118 of the ultrasound transducer 106. For ensuring a good fitting of the sizes of the central aperture 128 and the ultrasound transducer 106, the frame section 126 may comprise telescopic elements that allow for a flexible change in the size of the frame section 126. Furthermore, the frame section 126 may comprise a locking mechanism configured to lock the telescopic elements with respect to each other, thereby keeping the frame section 126 in the desired size once the fitting frame section size has been found.

As a further alternative to the embodiments with two membranes forming a closed hollow or only one membrane forming a hollow with an opening, the present disclosure also provides an embodiment of a stand-off cushion which comprises two (or more membranes) and at least one membrane of the two (or more) membranes has at least one opening.

For example, the stand-off cushion 204 of Fig. 3 may be modified so that the cover 244 comprises an opening. Analogously, the embodiment of Fig. 4 may be modified so that the foil 352 forming the top cover comprises an opening. The opening may be shaped like a slit or slot hole, for example. The opening may not be closed during usage of the stand-off cushion. As is the case for the open, upper surface of the first modified embodiment of the stand-off cushion 204', 304', the opening of the second modified embodiment of the stand-off cushion 204", 304" is located in the central aperture 128 of the frame section 126 when the second modified stand-off cushion 204", 304" is mounted to the stand-off holder 102. When the opening is not closed during usage and the size of the opening is chosen to be the same size as the size of the ultrasound transducer 106, i.e. the end surface of the end portion of the ultrasound transducer 106, reverberation artifacts in the ultrasound image may be avoided and at the same time the cushion filling is prevented from spilling during the usage of the second modified stand-off cushion 204", 304".

The opening allows an easy filling of the stand-off cushion with a filling material, such as a gel-like or viscous material. Given that the opening stays open and there is spillage when pressed, the cushion may be delivered as pre-gelled. Furthermore, analogously to the open upper surface of the first modified embodiment with only one membrane, the opening of the second modified embodiment dispenses with the need to apply a coupling agent on the upper surface of the stand-off cushion 204", 304", a coupling agent only has to be applied on the opposite, lower side of the stand-off cushion 204", 304".

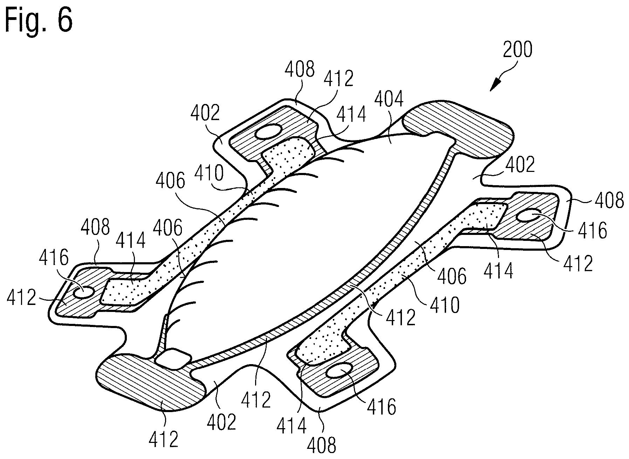

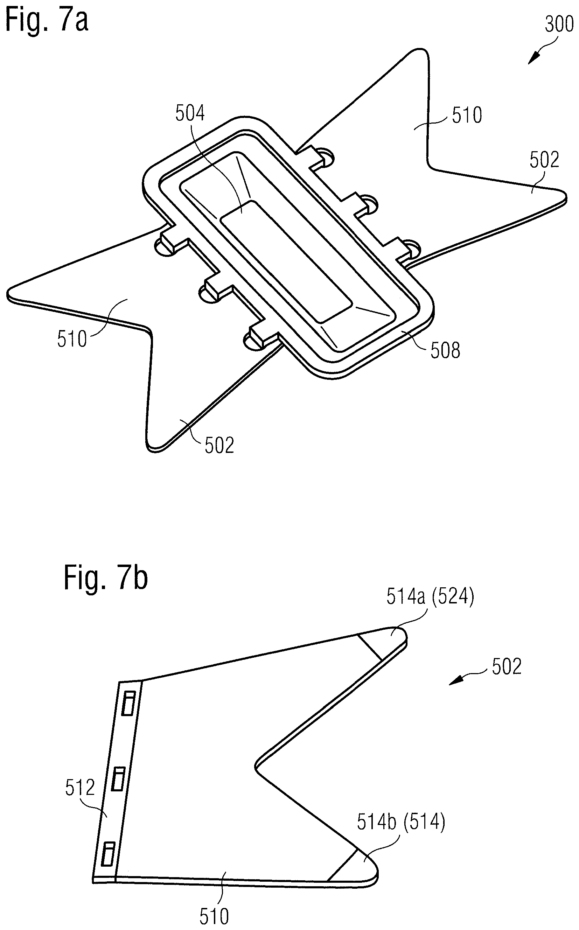

Figs. 6, 7a, and 7b of the drawings show two further embodiments of an ultrasound coupling system 200, 300 for coupling an ultrasound transducer to a body surface to be examined according to the present disclosure. In contrast to the ultrasound coupling system 100 discussed with respect to Figs. 1 to 5, the embodiments 200, 300 of Figs. 6 and 7a, b differ in that they do not comprise a stand-off holder 102. Instead, each of the stand-off cushions of the coupling systems of Figs. 6 and 7a, b may be directly, i.e. without a stand-off holder, coupled to the ultrasound transducer 106. In particular, each of the stand-off cushions of the coupling systems 200, 300 of Figs. 6 and 7a,b may be glued to the ultrasound transducer 106. More particularly, the stand-off cushion may be non-permanently glued to the ultrasound transducer 106 by means of at least one tab which comprises at least one adhesive region.

In the embodiment of Fig. 6, a central, flexible stand-off cushion 404 is surrounded by a tab 402. In Fig. 6, the central stand-off cushion 404 has the shape of an oval balloon, however, according to the present disclosure, the central stand-off cushion 404 may have any other three-dimensional shape, for example the shape of a bar or ingot. The tab 402 has a generally flat shape and comprises a base 406 completely surrounding the stand-off cushion 404 and extensions 408 protruding from the base 406. The extensions 408 comprise openings 416. One of the surfaces of the tab 402 comprises at least one adhesive region. In the embodiment of Fig. 6, the with respect to Fig. 6 upper surface comprises four adhesive regions 414, located at the tab 402 and covered by release liners 410, the latter extending along a long side of the oval-shaped stand-off cushion 404 and, at its opposing ends, being widened in a direction towards a respective extension 408 so as to extend into the respective extension 406. The widened portions form adhesive "points" 414, i.e. the adhesive regions 414, for tightly connecting the stand-off cushion to the ultrasound transducer.

The tab 402 is integrally formed with the central stand-off cushion 404. In particular, the tab 402 is integrally formed with the envelope of the stand-off cushion 404 by attaching an upper envelope layer with a lower envelope layer, preferably by welding the upper and lower envelope areas. The attached or welded areas 412 are schematically illustrated as line-hatched areas in Fig. 6. As is seen in Fig. 6, the upper and lower envelope layers are attached to each other along the outer circumference of the stand-off cushion 404, thereby forming a welded or attached area 412 for forming the closed hollow of the stand-off cushion 404. Furthermore, as the release liners 410, the attached or welded areas 412 extend along a long side of the oval-shaped stand-off cushion 404 and, at its opposing ends, extend further into the respective extension 406 than the release liners 410. In particular, in the embodiment of Fig. 6, the release liners 410 are provided on part of the welded areas 412 and are schematically illustrated as dot-hatched areas on welded areas 412. The welded areas 412 may also extend over the whole tab 402. The adhesive areas 414 may extend over the whole of the welded areas 412. The adhesive areas 414 may also extend over the whole tab 402, leaving however room for a thumb-sized gripping area on at least one end of the tab 402, preferably more. The openings 416 at the extensions 408 can optionally be used as mechanical fixation mechanism, where the cushion 404 is connected to a probe holder via corresponding extrusions or a cord receiving the openings 416, similar to the mounting openings 248. Alternatively, the openings 416 are removed.

The adhesive portions 414 may be provided in any manner known to the skilled person. For example, the adhesive portions 414 may be provided by an adhesive foil, wherein the adhesive foil may be a one-sided adhesive or a two-sided adhesive. As a further example, the adhesive portions 414 may also be provided by applying a glue on defined portions of the tab 402, in particular on defined portions of the welded areas 412 of the tab 402. As a further example, the envelope layer material may be adhesive such that the adhesive portions 414 may also be provided by the envelope layer itself. Since the tab 402 is made of the envelope material, the tab 402 is flexible. In particular, the tab 402 may be bent with respect to the central stand-off cushion 404 for fixing the stand-off cushion 404 to the ultrasound transducer. The fixing is removable. Thus, the stand-off cushion 404 may be removed from the ultrasound transducer without leaving any adhesive residue.

The material of the envelope and filling of the stand-off cushion 404 of Fig. 6 may be the same as the material of the envelope and filling of any of the stand-off cushions disclosed with respect to Figs. 1 to 5. Furthermore, the stand-off cushion may be configured as the stand-off cushions 204, 304 and modified stand-off cushions 204", 304" according to a second modification described with reference to Figs. 3 and 4, however without the frame 354 of Fig. 4 and the mounting holes 248 of Fig. 3, and provided that the upper and/or lower membranes laterally extend beyond the outer circumference of the stand-off cushion 404 so as to form the tab 402. For example, it is conceivable to modify the embodiment of Fig. 3 by lengthening the collar 246 of the envelope 212 so as to form the tab 402. In that case, there would be no need to provide a welded or attached area 412 on the tab 402, since the tab 402 comprises only one membrane. Instead, a welded or attached area only had to be provided at the interface between the envelope 212 and the cover 244.

In the embodiment of Figs. 7a and 7b, a central stand-off cushion 504 is surrounded by and connected to a rigid frame 508. For example, the central stand-off cushion 504 may be welded to the rigid frame 508. In the embodiment of Fig. 7a, the stand-off cushion 504 is bar- or ingot-shaped and the frame 508 is rectangular-shaped, however, according to the present disclosure, the central stand-off cushion 504 may have any other three-dimensional shape. The central stand-off cushion of Fig. 7a comprises an envelope which forms a closed hollow. The envelope may, for example, comprise an upper envelope layer and a lower envelope layer attached to each other by means of the frame 508. Two tabs 502 are pivotally connected to the frame 508, in particular to the long sides of the rectangular frame 508 . The two tabs 502 can be made of the same material as the frame 508. They can also be very flexible and made of a one-sided adhesive. They can further be made of the same material as the stand-off cushion envelope. As is seen in an exemplary manner in Fig. 7b for one of the tabs 502, an upper surface of each tab 502 comprises an adhesive region 510, which is covered by a release liner. In the embodiment of Figs. 7a and 7b, the adhesive region 510 covers the whole upper surface with the exception of a connection region 512 of the tabs 502 where the tabs 502 are configured to be connected to the frame 508 and an outer gripping portion 514 configured to be gripped for coupling and decoupling the stand-off cushion 504 to and from the ultrasound transducer. In the embodiment of Figs. 7a and 7b, each outer gripping portion 514 comprises two gripping portions 514a, 514b.

The material of the envelope and filling of the stand-off cushion 504 of Fig. 7 may be the same as the material of the envelope and filling of any of the stand-off cushions disclosed with respect to Figs. 1 to 5. Furthermore, the stand-off cushion may be configured as the stand-off cushions 204, 304 and modified stand-off cushions 204', 304' and 204", 304" according to the first and second modifications described with reference to Figs. 3 and 4, however with the frame 508 instead of the frame 354 of Fig. 4. The mounting holes 248 of Fig. 3 may be omitted or may be used for connecting the frame 508 to the cushion 204, 204', 204".

Thus, for example, the stand-off cushion 404, 504 of Figs. 6 and 7a may comprise an envelope which forms a hollow having an opening and being filled with a gel-like and/or relatively soft material, wherein the opening is located so that it faces the end surface of the ultrasound transducer in a state where the stand-off cushion 404, 504 is mounted to the ultrasound transducer by means of the tab 402, 502.

For example, the upper envelope layer of the stand-off cushion 404, 504 may comprise an opening. The opening may be shaped like a slit or slot hole. The opening may not be closed during usage of the stand-off cushion 404, 504. The size of the opening may be chosen to be substantially the same size as the size of the ultrasound transducer, i.e. the end surface of the end portion of the ultrasound transducer.

Furthermore, for example, the stand-off cushion 504 of Fig. 7a may only comprise the lower envelope layer instead of two envelope layers thereby forming a hollow that comprises at least one opening. The opening may be provided in an upper surface of the stand-off cushion being configured to be contacted by the ultrasound transducer, wherein the upper surface corresponds to the surface to be covered by the second membrane in an implementation with two membranes and a closed hollow. In that way, the ultrasound transducer when being fixed with respect to the cushion 300 by means of the tabs 502 may contact the open surface of the stand-off cushion 300, i.e. may contact the filling material of the stand-off cushion 300.

Furthermore, for example, each of the stand-off cushions of Fig. 6 and 7a may have an outer envelope made of or comprising an aromatic polyether polyurethane and the filling material may be an ultrasound gel or water.

The stand-off cushion 404 of Fig. 6 can be connected in a simple manner to an ultrasound transducer 106 by bending the tab 402 with respect to the cushion 404 in a direction towards the ultrasound transducer 102 and by gluing the tab 402 to the ultrasound transducer 106. Analogously, the stand-off cushion 504 of Figs. 7a, 7b can be connected in a simple manner to an ultrasound transducer 106 by pivoting the tabs 502 with respect to the cushion 504 in a direction towards the ultrasound transducer 106 and by gluing the tabs 502 to the ultrasound transducer 106. The embodiments of Figs. 6 and 7 have the advantage that the cushion 404, 504 and the tab 402 or tabs 502 can be adapted to the most diverse ultrasound transducer sizes and shapes.

The stand-off cushion according to the present disclosure is made of a highly flexible material, thereby being deformable in all three directions of the space. Typical dimensions of the stand-off cushion that will fit most ultrasound transducers according to any embodiment of the present disclosure may be 7 cm for the length dimension and 2 cm for the width dimension. For large ultrasound transducers, typical dimensions according to any embodiment of the present disclosure may be 7 cm for a length dimension and 4 cm for a width dimension. In a filled state, the dimensions may vary up to a few centimeters, depending on the internal pressure exerted on the envelope of the cushion and elasticity of the material. In addition, in the embodiments with a stand-off holder, the stand-off cushion is located at an outer side of the stand-off holder, thus being not limited in its deformation by any space constraints defined by the configuration of the stand-off holder. The same applies to the embodiments of Figs. 6, 7a and 7b due to the absence of a stand-off holder which might define any space constraints. Thus, the stand-off cushion may adapt to all kinds of surfaces and shapes thereby maximizing the contact surface of the ultrasound transducer with the body part to be examined. Further, the height of the stand-off cushion can be adjusted manually and flexibly by changing the pressure on the stand-off cushion. In that way, for example, the focus in the ultrasound image may be adapted dynamically.

## Claims

1. An ultrasound coupling system (200, 300) for coupling an ultrasound transducer (106) to a body surface to be examined, comprising
a flexible stand-off cushion (404, 504) comprising an envelope which comprises a flexible material, wherein the envelope forms a closed hollow which is filled with a liquid, gel-like and/or relatively soft material, and
a flexible tab (402, 502) connected to the stand-off cushion (404, 504) and having an adhesive region (414, 510) for coupling the stand-off cushion (404, 504) to the ultrasound transducer (106),
wherein the flexible tab (402) comprises an attached area (412) formed by an upper envelope layer and a lower envelope layer attached to each other for forming the closed hollow of the flexible stand-off cushion (404), or
wherein the ultrasound coupling system (300) comprises a frame (508) holding the stand-off cushion (504), wherein the flexible tab (502) is directly connected to the frame (508).

2. The ultrasound coupling system of claim 1, wherein the upper envelope layer and the lower envelope layer are attached at predefined areas where the upper envelope layer and the lower envelope layer extend beyond the flexible stand-off cushion (404).

3. The ultrasound coupling system of any one of claims 1 or 2, wherein the upper envelope layer and the lower envelope layer are attached by welding the upper envelope layer and the lower envelope layer.

4. Ultrasound measurement system for guiding an ultrasound transducer (106) comprising the ultrasound coupling system (200, 300) of any of claims 1 to 3.

## Patentansprüche

1. Ultraschall-Kopplungssystem (200, 300) zum Ankoppeln eines Ultraschallwandlers (106) an eine zu untersuchende Körperoberfläche, umfassend
ein flexibles Abstandskissen (404, 504), das eine Hülle umfassend ein flexibles Material umfasst, wobei die Hülle einen geschlossenen Hohlraum bildet, der mit einem flüssigen, gelartigen und/oder relativ weichen Material gefüllt ist, und
eine flexible Lasche (402, 502), die mit dem Abstandskissen (404, 504) verbunden ist und einen Klebeabschnitt (414, 510) zum Anbringen des Abstandskissens (404, 504) an dem Ultraschallwandler (106) aufweist,
wobei die flexible Lasche (402) einen Verbindungsbereich (412) umfasst, der durch eine obere Hüllschicht und eine untere Hüllschicht gebildet wird, die aneinander befestigt sind, um den geschlossenen Hohlraum des flexiblen Abstandskissens (404) zu bilden, oder
wobei das Ultraschallkopplungssystem (300) einen Rahmen (508) umfasst, der das Abstandskissen (504) hält, wobei die flexible Lasche (502) direkt mit dem Rahmen (508) verbunden ist.

2. Ultraschall-Kopplungssystem nach Anspruch 1, wobei die obere Hüllschicht und die untere Hüllschicht an vordefinierten Bereichen befestigt sind, an denen die obere Hüllschicht und die untere Hüllschicht über das flexible Abstandskissen (404) hinausragen.

3. Ultraschall-Kopplungssystem nach einem der Ansprüche 1 oder 2, wobei die obere Hüllschicht und die untere Hüllschicht durch Verschweißen der oberen Hüllschicht und der unteren Hüllschicht befestigt sind.

4. Ultraschallmesssystem zum Führen eines Ultraschallwandlers (106) umfassend das Ultraschall-Kopplungssystem (200, 300) nach einem der Ansprüche 1 bis 3.

## Revendications

1. Système de couplage ultrasonore (200, 300) destiné à coupler un transducteur à ultrasons (106) à la surface d'un corps à examiner, comprenant
un coussin d'écartement souple (404, 504) comprenant une enveloppe qui est réalisée dans un matériau flexible, dans lequel l'enveloppe forme une cavité fermée qui est remplie d'un matériau liquide, du type gel et/ou relativement souple, et
une languette flexible (402, 502) reliée au coussin d'écartement (404, 504) et comportant une zone adhésive (414, 510) pour fixer le coussin d'écartement (404, 504) au transducteur à ultrasons (106)
dans lequel la languette flexible (402) comprend une zone de fixation (412) formée par une couche d'enveloppe supérieure et une couche d'enveloppe inférieure fixées l'une à l'autre afin de former la cavité fermée du coussin d'écartement flexible (404), ou
dans lequel le système de couplage ultrasonore (300) comprend un cadre (508) supportant le coussin d'écartement (504), la languette flexible (502) étant directement reliée au cadre (508).

2. Système de couplage ultrasonore selon la revendication 1, dans lequel la couche d'enveloppe supérieure et la couche d'enveloppe inférieure sont fixées au niveau de zones prédéfinies où la couche d'enveloppe supérieure et la couche d'enveloppe inférieure s'étendent au-delà du coussin d'écartement flexible (404).

3. Système de couplage ultrasonore selon l'une quelconque des revendications 1 ou 2, dans lequel la couche d'enveloppe supérieure et la couche d'enveloppe inférieure sont assemblées par soudage de la couche d'enveloppe supérieure et de la couche d'enveloppe inférieure.

4. Système de mesure par ultrasons destiné à guider un transducteur à ultrasons (106), comprenant le système de couplage ultrasonore (200, 300) selon l'une quelconque des revendications 1 à 3.
